# EUROPEAN PATENT SPECIFICATION

(11) **EP 4 175 690 B1**
(45) Date of publication and mention of the grant of the patent: **05.11.2025**
(21) Application number: 21746148.2
(22) Date of filing: 01.07.2021
(51) Int. Cl.: A61L 29/04, A61M 27/00

(54) **FEMALE URINE DRAINAGE DEVICE**
URINDRAINAGEVORRICHTUNG FÜR FRAUEN
DISPOSITIF DE DRAINAGE D'URINE CHEZ LA FEMME

(30) Priority: 03.07.2020 GB 202010232
(43) Date of publication of application: 10.05.2023
(73) Proprietor: URO INNOVATIONS LIMITED, Exeter, EX1 1QT (GB)
(72) Inventor: HUNTER, Gary Francis, Exeter Devon EX2 8BP (GB)
(74) Representative: Craske, Stephen Allan
(86) International application number: PCT/IB2021/055910
(87) International publication number: WO 2022/003619

(56) References cited:
- EP-A1- 0 836 500
- GB-A- 2 528 466
- CLARIANT: "Mowiol", 1 January 1999 (1999-01-01) - 1999, pages 1 - 105, XP093249480, Retrieved from the Internet <URL:https://ia903101.us.archive.org> Mowiol manual>
- GOOD F MICHAEL ET AL: "Suppl. Material: Cross-species malaria immunity induced by chemically attenuated parasites", J CLIN INVEST, 1 July 2013 (2013-07-01), pages 3353 - 3362, XP093249280, Retrieved from the Internet <URL:https://dm5migu4zj3pb.cloudfront.net/manuscripts/66000/66634/JCI66634sd.pdf> DOI: 10.1172/JCI66634

## Description

### TECHNICAL FIELD OF THE INVENTION

This invention relates to female urine drainage devices.

### BACKGROUND

Intermittent self catheterisation of the bladder via the urethra is a widely performed procedure, which may, for example, be necessitated by operative procedures or spinal cord injuries where the patient loses the ability to control their bladder.

A ubiquitous urine drainage device comprises a flexible catheter tube which is manufactured from a soft thermoplastic material such as polyvinyl chloride. Such devices are widely used by both males and females, the female version being substantially shorter. Normally the catheter tubes have one or more openings at a proximal end which is inserted through the urethra into the bladder, and a connector at the opposite distal end is used to attach a drainage tube or urine collection bag. Such catheters have many advantage such as being soft and flexible, durable, and are capable of being coated with lubricating substances which become activated when wet.

A female urinary drainage device which is integrally moulded with an integral closure and hand grip has been manufactured for about two decades. The device is described in EP 0 836 500-A1 and has won awards for its innovative features. The unique hand grip enables female patients with poor hand dexterity to insert the device into their bladder more easily, and the integral closure with a unique opening and closing mechanism enables the user to decide, in her own time, when she is ready to drain. Another notable feature is that it was the first intermittent urinary drainage device to be moulded from cornstarch. The device is reusable for up to 24 hours, and is 100% biodegradable.

Compared with male and female drainage devices comprising a flexible catheter tube, the biodegradable moulded cornstarch product is relatively inflexible, which is an important requirement to allow the opening and closing mechanism to operate. Although biodegradable, they do not readily dissolve in water so that they are normally discarded in a bin, which can be inconvenient and embarrassing for the user.

### SUMMARY OF THE INVENTION

When viewed from one aspect the present invention proposes a one-piece integrally moulded female urine drainage device. The device has the features specified in claim 1 below.

The solubility of vinyl alcohol polymers depends largely on their amount of hydrolysis. A polymer with a level of hydrolysis between 85 to 89% dissolves easily at room temperature, while PVA that has high hydrolysis degree (98-99%) is not soluble at room temperature. The proposed device is water soluble above room temperature and can be flushed down a toilet while at the same time having sufficient stiffness and durability to retain the features which are much favoured by female users. Another significant feature is that the entire device can be flushed down a toilet without having to detach a non-soluble connector.

### BRIEF DESCRIPTION OF THE DRAWINGS

The following description and the accompanying drawings referred to therein are included by way of non-limiting example in order to illustrate how the invention may be put into practice. In the drawings:
Figure 1 is a general view of a female urine drainage device prior to insertion into the bladder;
Figure 2 is a similar view of the female urine drainage device which is in use to drain the bladder.

### DETAILED DESCRIPTION OF THE DRAWINGS

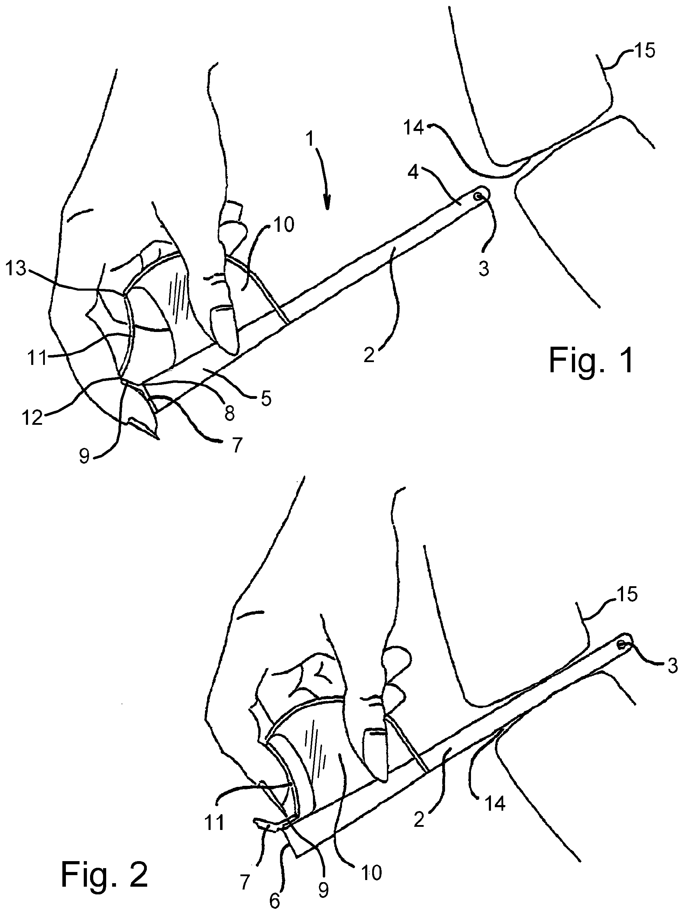

Referring firstly to **Fig. 1****,** the drainage device 1 has a tubular part 2 with one or more lateral inlet openings 3 at a proximal end 4. The opposite distal end 5 has a single outlet opening 6 **(****Fig. 2****),** which is disposed co-axially with the tubular part 2, and a closure 7 which is integrally joined to one edge of the outlet opening by a first hinge 8. The closure 7 is shaped to cover the opening 6 and includes an angularly-projecting extension arm 9. An integral axially-extending hand grip 10 projects from one side of the tubular part 1 adjacent to the distal end 5. A curved resilient
member 11 connects the closure extension arm 9 to the hand grip 10, being connected to the extension arm 9 by a second hinge 12 and to the hand grip 10 by a third hinge 13. All three of the hinges 8, 12 and 13 are web hinges which are integrally formed with the adjacent elements.

When the drainage device is in a first stable configuration as in **Fig. 1****,** the resilient member 11 urges the closure 7 against the outlet opening 6. As shown in the drawing, a female user can conveniently exert moderate pressure on the closure 7 by means of their index finger to ensure closure of the outlet opening whilst holding the hand grip 10 with the tubular part 1 in a comfortable position for insertion into the urethra 14. The closure 7 therefore effectively blocks the flowpath through the tubular part 1. When the tubular part has been inserted far enough for inlet opening 3 to lie within the bladder 15 as shown in **Fig. 2****,** the user can, at her convenience, slide her index finger onto the extension arm 9 and resilient member 11. The slight increase in pressure on these parts causes the closure 7 to flip into an open position permitting urine to drain from the outlet opening 6. When drainage is complete, with slight reverse movement of the index finger the closure 7 can be flipped back to the closed position to prevent urine from dripping from the opening 6 while the tubular part 2 is withdrawn from the urethra 14.

A preferred water soluble and suitably mouldable material is vinyl alcohol polymer, INS No. 1203, as manufactured by Distrupol, a Univar company. Vinyl alcohol polymer is a synthetic resin which is prepared by the polymerization of vinyl acetate, followed by partial hydrolysis of the ester in the presence of an alkaline catalyst. The physical characteristics of the product depend on the degree of polymerization and the amount of hydrolysis. Vinyl alcohol polymer has the chemical formula [CH2CH(OR)]ₙ where R = H or COCH₃, with the COCH₃ groups being randomly distributed throughout the molecule. Vinyl alcohol polymer has a wide range of uses, and is approved for use as a biomaterial in medical devices due to its proven biocompatible, nontoxic and noncarcinogenic properties. To touch and feel, the material can be as flexible as low density polyethylene (LDPE) or as rigid as polypropylene (PP). Injection mouldable grades have been developed with a melt flow rate as low as 1g/10 min and as high as 100g/10 min. On initial contact with water vinyl alcohol polymer forms a lubricating surface. On longer exposure the material dissolves to form acetic acid, which is incorporated into the natural biomass by naturally occurring microorganisms.

The solubility of vinyl alcohol polymers depends largely on their amount of hydrolysis. A polymer with a level of hydrolysis between 85 to 89% dissolves easily at room temperature, while PVA that has high hydrolysis degree (98-99%) is not soluble at room temperature. This difference in solubility is due to the hydrogen bonds between the vinyl alcohol chains which are greater when the degree of hydrolysis is high.

MonoSol of the USA produces a vinyl alcohol polymer under the trade name MonoPol which can be used to produce injection moulded products which dissolve when they contact water so that they can be dissolved away without leaving contaminated waste. The MonoPol family includes a variety of formulations which can be selected to work at different fabrication speeds and different temperatures. Moulding also allows structures to be customized by changing wall thickness and dimensional strength. MonoPol is available in four grades: cold water soluble (from about 10 degrees C) to very hot water soluble (above 70 degrees C). Each grade is available in a range of colours, and mechanical properties can be adjusted and customised for specific applications.

The female urine drainage device is particularly suitable for intermittent self catheterisation and has advantages over known devices:
- The entire product is water soluble, environmentally friendly and flushable.
- The user retains full control over urine drainage.
- The tubular part is self-lubricating on contact with water.
- The device is hypoallergenic.

## Claims

1. A one-piece integrally moulded female urine drainage device:
- a tubular part (2) having a distal end (5) and a proximal end (4);
- an inlet opening (3) at the proximal end;
- an outlet opening (6) at the distal end;
- a hand grip (10) projecting from the tubular part;
- a closure (7) for closing the outlet opening;
- a first hinge (8) connecting the closure to the tubular part;
- an opening-and-closing mechanism (9, 11) connected between the closure (7) and the hand grip (10) and which, in a stable configuration, urges the closure (7) against the outlet opening (6);
**characterised in that** the device is formed of a water soluble mouldable material which has the chemical formula [CH2CH(OR)]n where R = H or COCH3, with the COCH3 groups being randomly distributed throughout the molecule, and the water soluble mouldable material has a degree of hydrolysis from 98-99%;
wherein the one-piece female urine drainage device is of sufficient stiffness to enable a user to insert the tubular part (2) into their urethra whilst holding the device by the hand grip (10) and, once inserted, allows the user to manipulate the opening-and-closing mechanism (9, 11) to flip the closure (7) to an open position permitting urine to drain from the outlet opening (6).

2. A female urine drainage device according to claim 1 in which the opening-and-closing mechanism comprises an extension arm (9) on the closure (7).

3. A female urine drainage device according to claim 2 in which the extension arm (9) is connected to a resilient member (11).

4. A female urine drainage device according to claim 3 in which the extension arm (9) is connected to the resilient member (11) by a second hinge (12).

5. A female urine drainage device according to claim 3 in which the resilient member (11) is connected to the hand grip (10).

6. A female urine drainage device according to claim 5 in which the resilient member (11) is connected to the hand grip (10) by a third hinge (13).

## Patentansprüche

1. Einteilige, integral geformte Urindrainagevorrichtung für Frauen:
- ein röhrenförmiges Teil (2) mit einem distalen Ende (5) und einem proximalen Ende (4);
- eine Einlassöffnung (3) am proximalen Ende;
- eine Auslassöffnung (6) am distalen Ende;
- einen vom röhrenförmigen Teil abstehenden Handgriff (10);
- einen Verschluss (7) zum Verschließen der Auslassöffnung;
- ein erstes Scharnier (8), das den Verschluss mit dem röhrenförmigen Teil verbindet;
- einen Öffnungs- und Schließmechanismus (9, 11), der zwischen dem Verschluss (7) und dem Handgriff (10) angeordnet ist und den Verschluss (7) in stabiler Konfiguration gegen die Auslassöffnung (6) drückt;
**dadurch gekennzeichnet, dass** Die Vorrichtung besteht aus einem wasserlöslichen, formbaren Material mit der chemischen Formel [CH₂CH(OR)]n, wobei R = H oder COCH₃ ist, wobei die COCH₃-Gruppen zufällig im Molekül verteilt sind und das wasserlösliche, formbare Material einen Hydrolysegrad von 98-99 % aufweist;
Die einteilige Urindrainagevorrichtung für Frauen ist so steif, dass der Benutzer das röhrenförmige Teil (2) am Handgriff (10) in die Harnröhre einführen kann. Nach dem Einführen kann der Benutzer den Öffnungs- und Schließmechanismus (9, 11) betätigen, um den Verschluss (7) in eine geöffnete Position zu klappen, sodass Urin aus der Auslassöffnung (6) abfließen kann.

2. Eine Urindrainagevorrichtung für Frauen nach Anspruch 1, wobei der Öffnungs- und Schließmechanismus einen Verlängerungsarm (9) am Verschluss (7) umfasst.

3. Eine Urindrainagevorrichtung für Frauen nach Anspruch 2, wobei der Verlängerungsarm (9) mit einem elastischen Element (11) verbunden ist.

4. Eine Urindrainagevorrichtung für Frauen nach Anspruch 3, wobei der Verlängerungsarm (9) über ein zweites Scharnier (12) mit dem elastischen Element (11) verbunden ist.

5. Eine Urindrainagevorrichtung für Frauen nach Anspruch 3, wobei das elastische Element (11) mit dem Handgriff (10) verbunden ist.

6. Eine Urindrainagevorrichtung für Frauen nach Anspruch 5, wobei das elastische Element (11) über ein drittes Scharnier (13) mit dem Handgriff (10) verbunden ist.

## Revendications

1. Dispositif de drainage urinaire féminin monobloc moulé d'un seul tenant:
- une partie tubulaire (2) présentant une extrémité distale (5) et une extrémité proximale (4);
- une ouverture d'entrée (3) à l'extrémité proximale;
- une ouverture de sortie (6) à l'extrémité distale;
- une poignée (10) faisant saillie de la partie tubulaire;
- un bouchon (7) pour fermer l'ouverture de sortie;
- une première charnière (8) reliant le bouchon à la partie tubulaire;
- un mécanisme d'ouverture et de fermeture (9, 11) relié entre le bouchon (7) et la poignée (10) et qui, en configuration stable, pousse le bouchon (7) contre l'ouverture de sortie (6);
**caractérisé en ce que** le dispositif est constitué d'un matériau moulable hydrosoluble de formule chimique [CH₂CH(OR)]n où R = H ou COCH₃, les groupes COCH₃ étant répartis aléatoirement dans la molécule, et ce matériau moulable hydrosoluble présentant un degré d'hydrolyse de 98 à 99 %;
le dispositif de drainage urinaire féminin monobloc est suffisamment rigide pour permettre à l'utilisatrice d'insérer la partie tubulaire (2) dans son urètre tout en le tenant par la poignée (10) et, une fois inséré, de manipuler le mécanisme d'ouverture et de fermeture (9, 11) pour ouvrir le bouchon (7) et permettre ainsi à l'urine de s'écouler par l'orifice de sortie (6).

2. Dispositif de drainage urinaire féminin selon la revendication 1, dans lequel le mécanisme d'ouverture et de fermeture comprend un bras d'extension (9) sur le bouchon (7).

3. Dispositif de drainage urinaire féminin selon la revendication 2, dans lequel le bras d'extension (9) est relié à un élément élastique (11).

4. Dispositif de drainage urinaire féminin selon la revendication 3, dans lequel le bras d'extension (9) est relié à l'élément élastique (11) par une deuxième charnière (12).

5. Dispositif de drainage urinaire féminin selon la revendication 3, dans lequel l'élément élastique (11) est relié à la poignée (10).

6. Dispositif de drainage urinaire féminin selon la revendication 5, dans lequel l'élément élastique (11) est relié à la poignée (10) par une troisième charnière (13).
